# EUROPEAN PATENT SPECIFICATION

(11) **EP 0 842 217 B1**
(45) Date of publication and mention of the grant of the patent: **08.01.2003**
(21) Application number: 96926560.2
(22) Date of filing: 24.07.1996
(51) Int. Cl.: C08J 7/12, A61L 27/00, C08L 67/04

(54) **A PROCESS OF SURFACE ACTIVATION OF BIOCOMPATIBLE AND BIOABSORBABLE ALIPHATIC POLYESTERS AND POLYESTERS THUS ACTIVATED**
VERFAHREN ZUR AKTIVIERUNG DER OBERFLÄCHEN VON BIOVERTRÄGLICHEN UND BIOABSORBIERBAREN ALIPHATISCHEN POLYESTERN UND SO AKTIVIERTEN POLYESTERN
PROCEDE D'ACTIVATION DE SURFACE DE POLYESTERS ALIPHATIQUES BIOCOMPATIBLES ET BIOABSORBABLES ET POLYESTERS ACTIVES PAR CE PROCEDE

(30) Priority: 28.07.1995 IT VR950068
(43) Date of publication of application: 20.05.1998
(73) Proprietor: SANITARIA SCALIGERA S.P.A., 37127 Avesa (IT)
(72) Inventor: ZUCCATO, Alessandro, I-37123 Verona (IT); PEREGO, Gabriele, I-10015 Ivrea (IT); CELLA, Gian, Domenico, I-28100 Novara (IT)
(86) International application number: IT9600149
(87) International publication number: WO97005193

(56) References cited:
- EP-A- 0 358 819
- EP-A- 0 558 965
- GB-A- 2 141 435
- GB-A- 2 167 665
- US-A- 5 236 563

## Description

The invention relates to surface-activated copolymers of lactic acid and ε-caprolactone and to a procedure for preparation thereof.

In recent times homo- and copolymers of lactic acid have been the subject of growing attention regarding their potential applications, in part already put into practice, in the biomedical sector.

Such applications, which take advantage of the biocompatibility and biodegradability of these materials, comprise among other things the manufacture of prosthetic implants, suture threads, microspheres for vehicling of principal active ingredients.

A relative advantage of these materials is that of eliminating the need for a second surgical intervention, sometimes necessary for removing the implant, thanks to the gradual biodegradability which favours regeneration of the pre-existing tissues.

This advantage has been exploited, for example, in the preparation of bioabsorbable suture threads using homo- and copolymers of glycolic acid, commercially known as DEXON (mark of the company Davis and Jeck) and VICRYL (mark of the company Ethicon). In more recent times commercialisation has begun of the first products for the orthopaedic sector, known as BIOFIX (mark of Bioscience Oy) and ORTHOSORB (mark of the company Johnson and Johnson).

A field of use of bioabsorbable materials which has great use potential is the preparation of vascular grafts. Implants at present available, often made in DACRON (a material provided by the company Du Pont of Nemurs), as they are not bioabsorbable, lack in some important requisites, such as for example a complete absence of thrombogenicity of the internal wall, or elastic properties which remain unaltered over time [New Polymeric Mater, 1 (2), 111-126 (1988)]. These limitations become important especially when prostheses are below 6mm diameter, which often leads during surgery to implant of autologous veins to repair small-caliber arteries, with all the problems deriving there-from.

The development of a biodegradable vascular prosthesis, which would thus promote the regeneration of pre-existing tissue, is of great interest and is at present the object of many studies for example, see Colloid Polym. Sci 264, 854-858 (1986), but is also hindered by the problem of thrombogenicity for small calibers.

Thrombotic formation on the internal wall of the prosthesis happens especially during the initial implant phase, that is before the wall has been covered with endothelial cells, for which reason a biocompatible and bioabsorbable material for the manufacturing of a vascular graft would have a relevant advantage if it could have surface alterations aimed at accelerating the endothelialisation process.

The copolymers of lactic acid (L-lactic) and ε-caprolactone, developed in 1975 by Gulf Oil see US Patent US-A-4,057,537 (Sinclair) are very interesting materials for various biomedical uses, among which the realisation of nerve guides Biomaterial 15 (3), 189-193 (1994), J.Mater Sci. Mater in Medicine 4, 521-525 (1993), suture threads and bioabsorbable vascular prostheses.

The interest in these materials is linked, other than for their biocompatibility and bioabsorbability, to their excellent elastic properties (Polymer Bulletin 25, 335-341 (1991)) which are stable over time, especially for copolymers with about equal components of two comonomers (Makromol Chem 194, 2463-2469 (1993)).

These materials, on the other hand, have the drawback as far as biomedical use is concerned of having a low hydrophilicity, which reduces their wettability and the adherence of the organism cells, namely the endothelial cells. For the poly (L-Lactide-co-ε-caprolactone) at 50/50 weight, for example, the wetting angle measured with distilled water in air at 23°C is 82°, thus very high.

GB-A-2,141,435 discloses a method based on irradiation or hydrolytic treatments to control the physical properties of structural surgical elements made from bioabsorbable polymer materials so that the rate of strength loss and degradation in vivo is alterated to achieve disintegration into fragments suitable for removal from or passage through the body without the need for waiting the material is absorbed.

EP-A-0 358 819 deals with an artificial skin or bandage which is formed from an elastomeric layer which is surfaced by a fibrous wound contacting layer which is first formed into a fabric and subsequently degraded by heat hydrolysis or other degradative treatment into a form which could no longer be formed into a fabric but which forms an idea bioadsorbable wound contact surface in which damage to the newly formed, fragile epidermus is minimized.

US-A-5 236 563 discloses a method of treating a bioabsorbable polymer by exposing the polymer to a gas plasma for a sufficient time to increase the hydrophobility and/or crosslinking density of the surface layer.

An aim of the present invention is to provide a process aimed at surface modification of a material based on one or more of the copolymers of lactic acid and ε-caprolactone such as to bring about a substantial increase in surface hydrophilicity and, consequently, a significant lowering of the wetting angle measured with water at 23 degrees Celsius in air. This can in many cases favour cellular adherence to bioabsorbable medical prostheses realised using these materials, and thus surface-activated, such as for example endothelialisation processes in vascular grafts.

A further aim of the present invention is that said treatment is also aimed at reducing autoadhesiveness of said material in order to achieve considerable advantages in the manipulation and use of films, medical prostheses and the like.

According to a first aspect of the present invention a process for the surface treatment of biocompatible and bioabsorbable aliphatic polyesters selected from a group formed by homo and copolymers derived from L-lactide, D-lactide, D,L -lactide, racemic D,L-lactide, meso D,L-lactide, ε-caprolactone, glycolide, δ-valerolactone, p-dioxanone, preferably copolymers of L-lactide, D- lactide, racemic D,L-lactide, meso D,L-lactide with ε-caprolactone used for the production of biomedical products, is characterised in that said process consists in carrying out an acid or base hydrolysis at temperatures comprised between 0°C and the softening temperature of the material and/or a treatment by cold plasma, said surface treatment increasing the surface hydrophilicity and lowering the wetting angle measured with water at 23°C in air and wherein the hydrolytic treatment does not induce any reduction in the mechanical properties of the products.

Claims 2 to 21 disclose advantageous ways of carrying out the above described process.

Advantageously, the process of hydrolysis is conducted in the presence of an acqueous solution of a base or an acid to accelerate surface reaction, inasmuch as excessively long processes of activation can induce a reduction in molecular weight and thus in the mechanical properties of the material.

At present preferred is the use of an acqueous solution of a base which constitutes a preferential condition inasmuch as it has demonstrated greater activity.

Suitable bases for obtaining watery solutions suited to the aim are, for example, strong alkalis, such as LiOH, Ba(OH)₂, Mg(OH)₂, NaOH, KOH, Na₂CO₃, Ca(OH)₂ and the weak bases, such as for example NH₄OH and the ammines such as methylamine, ethylamine, diethylamine and dimethylamine.

Acids suitable for surface hydrolysis treatments can be chosen, for example, from among HCl, HClO₃, HClO₄, H₂SO₃, H₂SO₄, H₃PO₃, H₃PO₄, HI, HIO₃, HBr, lactic acid, glicolic acid.

Surface activation by means of hydrolysis can be conducted at temperatures preferably comprised between 0 °C and the material softening temperature.

In the case of the 50/50 poly(L-lactide-co-e-caprolactone), suitable temperatures are, for example, comprised between 5° and 80°C, preferably between 30° and 50°C.

According to another aspect of the present invention a biocompatible and bioabsorbable aliphatic polyester selected from a group formed by homo and copolymers derived from L-lactide, D-lactide, D,L -lactide, racemic D,L-lactide, meso D,L-lactide, ε-caprolactone, glycolide, δ-valerolactone, p-dioxanone preferably copolymers of L-lactide, D- lactide, racemic D,L-lactide, meso D,L-lactide with ε-caprolactone, is characterized in that the surface of said polymer is provided with a plurality of microporosities which increase the surface hydrophilicity and reduce the wetting angle measured with water at 23°C in air without inducing any reduction in the mechanical properties of said aliphatic polyester.

Claims 23 and 24 disclose advantageous forms of embodiment of the above described polyester.

Surface hydrolysis treatment is followed by careful washing to remove all traces of acid or base.

This surface hydrolytic treatment has been found to:
- produce a relevant surface change accompanied by a lowering of the wetting angle measured with water at 23°C in air, which in the case of poly(L-lactide-co-ε-caprolactone) at 50/50 weight passes from 82° to 30°;
- modify morphologically the surface of the material with the creation of numerous microporosities, useful in cell seeding processes, such as for example endothelialisation;
- substantially reduce autoadhesivity of the material, whose shear detachment force changes from an initial 10Kg/cm to 0 Kg/cm following colonisation, with considerable advantages in manipulation and use of films, medical prostheses, etc.;
- unexpectedly, the viscosity inherent in the copolymer during the process of activation does not undergo appreciable variation, such as to reduce its mechanical properties.

According to a preferred form of embodiment of the present invention, the process is carried out using homo- or copolymers of lactic acid, preferably the copolymers of lactic acid and e-caprolactone.

These copolymers can be obtained, as is known, by means of polymerisation by ring opening polymerisation of the cyclic dimer ring of lactic acid (L-lactic, in the case of L-lactic acid) and ε-caprolactone, at temperatures generally comprised between 100°C and 200°C and in the presence of a suitable catalyst, such as for example Sn octanoate, SnCl₂, Al(iOPr)₃, etc.

The copolymers of lactic acid and ε-caprolactone are suitable for working with the common methodologies used in heat-forming of plastic materials, such as for example, extruders, injection-moulders and so on.

These copolymers are also soluble in various organic solvents, among which, for example, methyl acetate, ethyl acetate, methylene choride, chloroform, etc., for which reason many manufactured products, for example canicular prostheses, can be obtained by exploiting this solubility, with known processes, such as for example deposition, spreading, etc,

Plasma treatment can be carried out both in the presence of a reactive gas, for example air, Ar, O₂ with the formation of surface activation of oxygenate type, such as -OH, - CHO, - COOH.

Surface treatment, whether hydrolytic or with plasma, can remain unaltered or can be followed by further chemical modifications. Thus, for example, the COONa groups generated by a base hydrolysis can be subsequently converted into COOH groups by treatment with strong mineral acids.

Further, the surface freeing of alcoholic groups by means of a hydrolysis process can be followed by reaction by means of the addition of a compound provided with functional group or groups able to react with surface alcoholic groups, such as for example by means of the addition of an anhydride such as succinic anhydride, with the conversion of -OH groups into -O-CO-CH₂-CH₂-COOH groups.

In a further aspect of the present invention, prosthetic implants are provided which are realised with homo and copolymers of lactic acid, surface-activated for obtaining biocompatible and biodegradable materials. An example thereof is constituted by a partially bioabsorbable vascular prosthesis.

The process according to the invention is described in more detail in the following, with reference to specific examples of practical realisation.

### Example 1

### Preparation of poly (L-lactide-co-ε-caprolactone)

L-lactide was synthesised and recrystallised 3 times by methyl anhydrous isobutyl ketone, dried under vacuum at 45°C for 24 hours and kept in a nitrogen atmosphere. ε-caprolactone, provided by Fluka, was dried on CaH₂, distilled at low pressure just before use and was kept under nitrogen. Hexane, provided by Farmitalia Carlo Erba, was distilled on LiAlH₄ and kept under nitrogen.

200 g of L-lactide, 200 g (186ml) of ε-caprolactone and an stirring magnet were placed in a preferably silanised (with Si(CH₃)₂Cl₂) 500ml two-neck flask. The flask was immersed in an oil bath whose temperature was gradually brought to 130°C, homogenizing the system as soon as made possible by the fusion of the lactide, by means of electromagnetic stirring.

A solution of 1.19g of Sn octanoate in hexane was prepared separately, up to a volume of 10ml, from which a measure of 2.13 ml was removed, which was equal to a content of 0.254g of Sn octoanate (having a catalysing function), which was then added to the monomer mixture. About 45 minutes after addition of the catalyst, whose quantity corresponded to a molar monomer/catalyst rapport of 5000, the electromagnetic stirring of the mixture became ineffective due to the system viscosity.

The reaction was protracted for 24 hours, thus obtaining an elastic transparent material having an inherent viscosity measured in chloroform at 25°C of 3.80dl/g and a residual monomer content of 2.3%, as estimated by TGA (thermogravimetric-isotherm analysis at 150°C for 60 minutes).

### Example 2

### Preparation of discs in 50/50 poly(L-lactide-co-ε-caprolactone)

A 10x10cm steel template was used, located between two Mylar sheets, in which 8 g of poly (L-lactide-co-ε-caprolactone) at 50/50 weight were placed.

Pressing was performed with three preheating minutes and 2 compression minutes at 50Kg/cm at a temperature of 160°C. Thus copolymer sheets were produced, having a thickness of 500 micron, from which 22mm diameter discs were die-cut. These discs exhibited a wetting angle with water, measured at 23°C in air, of 82°. The inherent viscosity of the material, determined in chloroform at 25°C, at a concentration of 0.2 g/dl, by Ubbelohde friction-tube viscometer, was 3.04 dl/g.

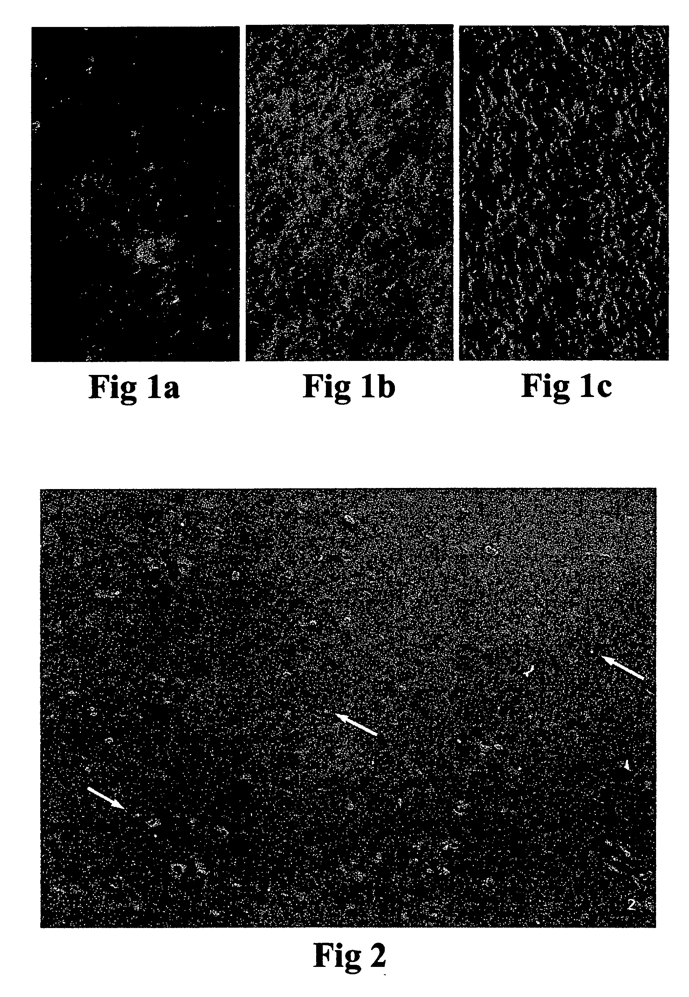

Figure 1a is a microphotograph obtained by scanning electron microscope (SEM) at a magnification of 1500x, which illustrates the surface morphology of a disc in non-activated poly(L-lactide-co-ε-caprolactone) prepared as above-specified.

### Example 3

### Activation by means of base hydrolysis at 40°C

80ml of dilute solution of NaOH at 5% were placed in a 100ml flask, and a disc of 50/50 poly (L-lactide-co-ε-caprolactone) having a thickness of 500 micron and a diameter of 22mm was prepared, as described in example 1. The flask was immersed in a thermostated water bath at 40°C, and stirred by an electromagnet.

These conditions were maintained for 18 hours, then the alkaline solution was removed and the whole was repeatedly washed with distilled water for four hours. The discs of the material thus activated exhibited a wetting angle with water of 30°, measured at 23°C in air. The inherent viscosity of the material, determined in chloroform at 25°C, at a concentration of 0.2g/dl was 3.00dl/g.

### Example 4

### Activation by means of base hydrolysis at 20°C

80 ml of dilute NaOH at 5% were placed in a 100ml flask and a 50/50 poly(L-lactide-co-ε-caprolactone) disc having a thickness of 500 micron and a diameter of 22mm was prepared, as described in example 1. The flask was kept at a temperature of 20°C, slowly stirring the contents by means of an electromagnet. These conditions were maintained for 18 hours, then the alkaline solution was removed and numerous washes with distilled water were carried out over a period of 4 hours.

The discs of material thus activated exhibited a wetting angle with water of 48°, measured at 23°C in air. The inherent viscosity of the material, determined in chloroform at 25°C at a concentration of 0.2g/dl was 3.02dl/g.

Figure 1b is a microphotograph obtained by scanning electron microscope (SEM) at a magnification of 1500x, which illustrates the surface morphology of a disc in poly(L-lactide-co-e-caprolactone) activated by base hydrolysis with NaOH according to the present example.

### Example 5

### Conversion of -COONa surface groups into -COOH groups.

80 ml of 3% HCl solution were placed in a 100ml flask and a 50/50 poly(L-lactide-co-ε-caprolactone) disc having a thickness of 500 micron and a diameter of 22mm was introduced, as described in example 3. The flask was kept at a temperature of 20C, slowly stirring the contents by means of an electromagnet for 1 hour, then the acid solution was removed and numerous washes with distilled water were carried out over a period of 4 hours.

The discs of material thus activated exhibited a wetting angle with water of 47°, measured at 23°C in air.

Figure 1c is a microphotograph obtained by scanning electron microscope (SEM) at a magnification of 1500x, which illustrates the surface morphology of a disc in poly(L-lactide-co-ε-caprolactone) activated by base hydrolysis with NaOH and acid hydrolysis by HCI.

### Example 6

### Conversion of -COONa surface groups, into -COOH groups

80 ml of 3% HCl acqueous solution were placed in a 100ml flask and a 50/50 poly (L-lactide-co-e-caprolactone) disc was introduced, as described in example 4. The flask was immersed in a thermostatised water bath at a temperature of 20°C, slowly agitating the contents by means of an electromagnet for 1 hour, then the acid solution was removed and numerous washes with distilled water were carried out over a period of 4 hours. The discs of material thus activated exhibited a wetting angle with water of 70, measured at 23°C in air.

### Example 7

### Activation by means of cold plasma.

A parallel flat plate Gambetti plasma treatment reactor with capacitative coupling was used. The radiofrequency the machine operated at was 13.6 Mhz.

Activation was carried out on discs of 50/50 poly(L-lactide-co-ε-caprolactone) having a thickness of 500 micron and a diameter of 22mm, prepared as described in Example 1.

These discs were applied with plasmas of air, oxygen and argon at a temperature of 20°C, at 20 and 50 Watts, for times of 30 and 60 seconds. The copolymer discs thus activated exhibited wetting angles with distilled water in air comprised between 31° and 55°, as reported in Table 1 hereinbelow.

It can be observed that the variation in treatment times, from 30 seconds to one minute, had a relevant effect only in the case of the low-potential plasmas used (20W), while with 50W the surface activation with the plasmas used seems to have reached a limit level after 30 seconds of treatment.

In the case of air, 50W potential plasma activated the surface of the material to a greater degree (32°-31°), with respect to 20W plasma (54.5, 47). In the case of oxygen and argon plasmas no significant differences were observed between the different potentials.

Among the experimental conditions adopted, therefore, a 50W potential air plasma produced the greatest activation. This can be explained by the fact that the inert part of air, especially nitrogen, generates numerous radicals on the surface of the material, which is followed by a reaction with oxygen molecules and the formation of activated groups of oxygenate type such as -OH, -CHO, -COOH.

**TABLE 1**

| Surface activation of 50/50 poly (L-lactide-co-ε-caprolactone) discs with cold plasma. | | | | |
|---|---|---|---|---|
| SAMPLE | GAS PLASMA | POTENTIAL (W) | TIME (SEC.) | WETTING ANGLE. |
| 1 | air | 20 | 30 | 54,5 |
| 2 | air | 20 | 60 | 47 |
| 3 | air | 50 | 30 | 32 |
| 4 | air | 50 | 60 | 31 |
| 5 | oxygen | 20 | 30 | 52 |
| 6 | oxygen | 20 | 60 | 44,5 |
| 7 | oxygen | 50 | 30 | 47 |
| 8 | oxygen | 50 | 60 | 55 |
| 9 | argon | 20 | 30 | 55 |
| 10 | argon | 20 | 60 | 46 |
| 11 | argon | 50 | 30 | 47 |
| 12 | argon | 50 | 60 | 47 |

### Example 8

### Measures of Material Autoadherence

We proceeded as in examples from 3 to 7 and the copolymer was successfully compression molded using a 5x5cm steel template having a thickness of 1mm, located between two Mylar® sheets, wherein were placed 3g of 50/50 poly(L-lactide-co-ε-caprolactone).

The compression molding was executed with 3 minutes of preheating and 2 minutes of compression at 50Kg/cm at a temperature of 160°C. Sheets of copolymer were thus prepared, having a thickness of 1mm, from which strips of 50x10x1mm were cut.

The test pieces for the autoadherence measurements were produced by superposing by 10mm the ends of two copolymer strips. The measurement of the shear force was effected immediately after pressing by using an Instron model 4501 dynamometer at a temperature of 23°C and in relative humidity of 50%. This calculation, effected at a velocity of 200mm/min., provided the force needed to cause shear detachment of the two surfaces, equal to 10Kg/cm for the non-modified copolymer.

The strips of surface-activated poly(L-lactide-co-ε-caprolactone) did not give rise to any adherence in the above-reported conditions.

Figure 2 is a SEM 100x microphotograph illustrating the surface morphology of a non-activated poly(L-lactide-co-ε-caprolactone) disc and seeded with endothelial cells from a microcirculation of human fatty tissue: 4 days of culture.

Note the presence of few cells having a prevalently rounded shape (some of which are indicated by arrows).

### Example 9

### Cell Culture on non-activated discs.

Discs of 50/50 poly(L-lactide-co-ε-caprolactone) having a thickness of 500 micron and a diameter of 22mm were subjected to sterilisation with ethylene oxide and kept in sterile containers up to the moment of cell seeding.

For the seeding human endothelial cells were used, isolated from the microcirculation of human fatty tissue, removed from the abdominal wall of patients subjected to surgical operation for abdominal aneurysm.

Variable quantities (10-20g) of fatty tissue were snipped using small scissors and transferred into an Erlemayer flask containing Collagenase Type 1 0.2% (SIOGMA - St. Louis Missouri USA) in PBS (Phosphate Buffer Saline pH 7.4).

After an incubation period of 30 minutes at 37°C, constantly shaken, the floating adipocytes were eliminated and the residual cellular suspension centrifuged for 10 minutes at 200 x g.

The resulting pellet was re-suspended in PBS (Phosphate Buffer Saline pH 7.4) containing 0.1% of BSA (Bovine Serum Albumin fraction V IgG free, Low endotoxin) (SIGMA) and the cellular suspension obtained was subjected to filtration through a nylon filter having a porosity of about 200 micron. After a further wash and centrifugation the pellet was re-suspended in 2ml of PBS/BSA and delicately layered on 8ml of Percoll 45% (mark of the company SIGMA) in PBS (280mOsm) and centrifuged at 1500 x g for 20 minutes.

The microvascular endothelial cells were recovered in a milky layer on the surface of the Percoll (SIGMA). After additional washes, the microvascular cells were re-suspended in Medium 199 25 MM Hepes (GIBCO BRL, Paisley, Scotland) containing 20% FCS (Foetal Calf Serum) (GIBCO BRL), 50g/ml ECGF (Endothelial Cell Growth Factor( (SIGMA), 100 U/ml heparin (SIGMA), 100 U/ml of penicillin (GIBCO BRL) and 100g(ml of streptomycin (GIBCO BRL) and subjected to hemocytometer count and Trypan Blue Stain exclusion test (Flow Laboratories, Irvine, Scotland) for evaluation of cell vitality.

All the operations described were carried out in conditions of total sterility under a laminar flow hood, and using sterile single-use glass and plastic materials. The poly(L-lactide-co-ε-caprolactone) discs were transferred, after repeated washes in sterile PBS (Phosphate Buffer Saline pH 7.4) in adequate size Cell Wells 12 well plate, well diameter 22 mm - CORNING, New York USA). On each disc, lying at the bottom of the wells, from 1 to 2 x 10 cells per cm were seeded in about 3ml of complete Medium 199 (SIGMA).

The culture was left for 4 to 6 days in an incubator at 37°C in a 5% CO₂ atmosphere.

The discs were kept 4 days under culture and then fixed in 2.5% glutaraldehyde (BAKER, Deventer, Holland) in cacodylate buffer (CARLO ERBA, Milan) and 1% osmic acid anhydrite (BAKER) in the same buffer, dehydrated in a growing series of ethanol (CARLO ERBA) and air-dried. Then they were subjected to analysis by means of scanning electron microscope.

On non-activated lactic acid copolymer discs only rare cells were visible, not numerically evaluable, and with a prevalence of rounded shapes (indicating poor adherence to substrate).

### Example 10

### Cell culture on NaOH activated discs.

Isolation of the endothelial cells and the cell culture were performed using the modalities described in example 9 above.

The SEM analysis of the lactic acid copolymer discs subjected to base hydrolysis enable observation of the adherence of a certain number of endothelial cells, not exactly quantifiable, which, al the same, exhibited aspects of better adherence to the substrate (flat cells).

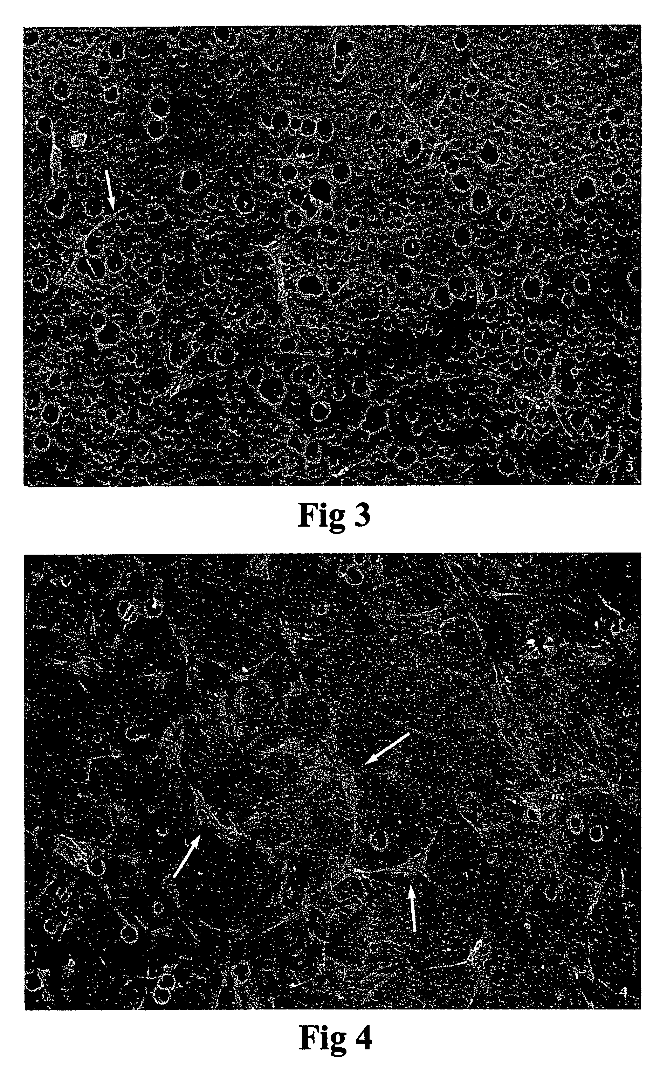

Figure 3 is an SEM X 100 microphotograph illustrating the surface morphology of a poly(L-lactide-co-ε-caprolactone) disc activated with NaOH and seeded with endothelial cells from the microcirculation of human fatty tissue: 4 culture days.

The microphotograph shows the presence of flat cells (indicated by arrows in figure 3).

### Example 11

### Cell culture on discs activated with NaOH/HCl.

Isolation of the endothelial cell and the cell culture was achieved using the modalities described in example 9. The SEM analysis of the discs subjected to base hydrolysis and further activated with HCl, allowed identification of a relevant number of endothelial cells (approximately 8 x 103/cm) with flat-spindled morphology, which is an indication of good adherence to the substrate.
Figure 4 is a SEM X 100 microphotograph showing the surface morphology of a disc in poly(L-lactide-co-ε-caprolactone) activated with NaOH and HCl and seeded with endothelial cells from the microcirculation of human fatty tissue: 4 culture days. The presence of numerous flat cells can be observed (indicated with arrows in the figure).
The results of the surface activation of 50/50 p(LLA-CL) discs are reported in Table 2.

**TABLE 2**

| Sample | reactive base | reactive acid | temperat. of wetting angle viscosity | | |
|---|---|---|---|---|---|
| | | | tr'tm'nt(°C) | (°) | iner. (dl/g) |
| I | - | - | - | 82 | 3,04 |
| II | NaOH 5% | - | 40 | 30 | 3,00 |
| III | NaOH 5% | HCl 3% | 20 | 47 | - |
| IV | NaOH 5% | - | 20 | 48 | 3,02 |
| V | NaOH 5% | HCl 3% | 20 | 70 | - |

### Example 12

### Cell culture on plasma: air 50W 1' surface activated discs.

Isolation of the endothelial cells and the cell culture were achieved using the modalities for example 6.

The SEM analysis of the lactic acid copolymer discs subjected to activation by oxygen at 50W for 1' evidenced a semiconfluent single layer of cells with prevalently spindled morphology.

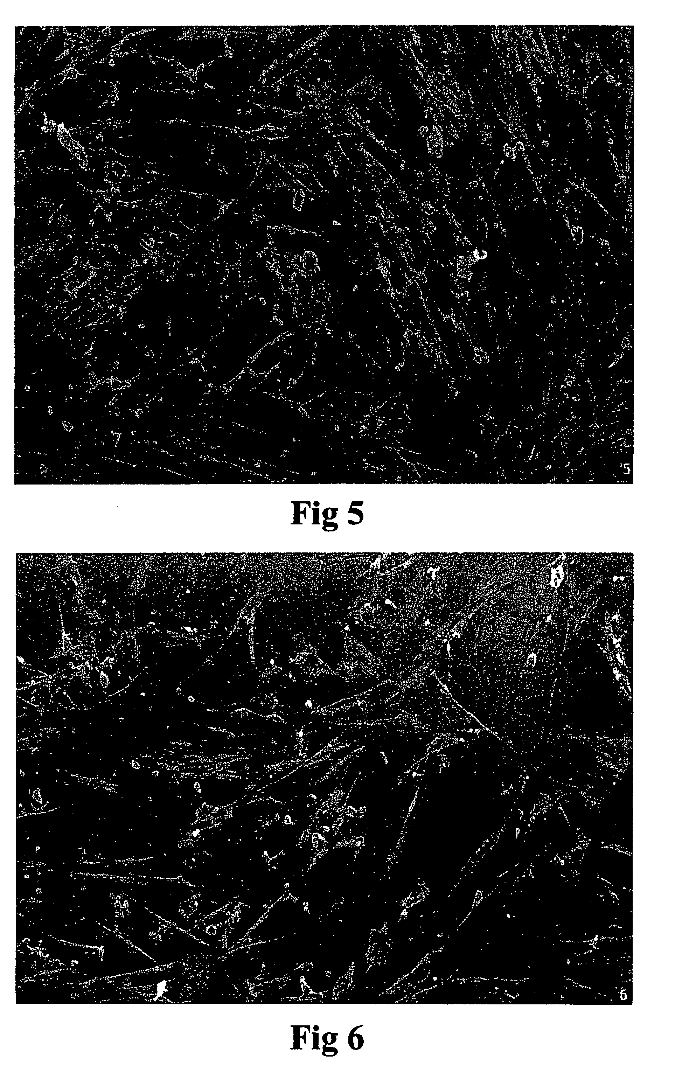

The cells appear particularly flat, a characteristic which permits sight, in transparency, of both the cytoplasmic organoids and the nucleus and sometimes even the nucleolus - see photograph of figure 5.

### Example 13

### Cell culture on plasma: O₂, 50W 30" surface activated discs.

Isolation of the endothelial cells and the cell culture were achieved using the modalities for example 6.

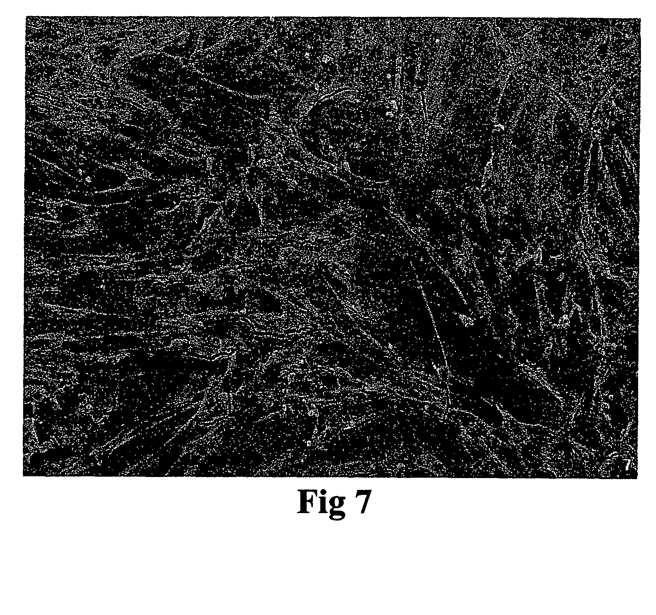

The SEM analysis of the lactic acid copolymer discs subjected to activation by oxygen at 50W for 30" evidenced a semiconfluent single layer of cells with prevalently elongate and flattened morphology. In some zones the cells appear less frequent and relatively large areas of polymer are left uncovered- see photographic reproduction of figure 6.

### Example 14

### Cell culture on plasma: Ar, 50W, 1' surface activated discs.

Isolation of the endothelial cells and the cell culture were achieved using the modalities for example 6.

The SEM analysis of the lactic acid copolymer discs subjected to activation by argon at 50W for 1' evidenced a semiconfluent monostrate of spindled and particularly flat cells - see photograph of figure 7.

This characteristic possibly represents the morphological equivalent of a more tenacious cell adherence to the non-activated substrate.

### Example 15

### Preparation of a partially bioabsorbable vascular prosthesis

the prosthesis was prepared with poly(L-lactide-co-ε-caprolactone) 50:50 by weight, synthesized as described in the example 1. A sample of 8.0 g of this copolymer was dissolved in anhydrous methyl acetate, to a volume of 100 ml. A rotating glass bar, fixed at one end in a mechanical stirrer and having a diameter of 6.0mm, was immersed in the solution. Tha bar was then slowly pulled out, so obtaining a uniform coating and the solvent was then evaporated at room temperature, with a gentle air stream. After having turned upside the glass bar, the operation was repeated to get a second layer and so on to prepare a prosthesis having the desired thickness, of 300 micron. The prosthesis, having a length of 10 cm, was dried under vacuum at room temperature and kept under nitrogen.

Alternatively the prosthesis was obtained by operating with an extruder having a screw with a length of 30 cm. The extrusion was performed at a speed of 40 rpm and at temperatures of 130°C, 150°C, 165°C, 170°C, 140°C, respectively from the first zone to the head of the extruder. The desired wall thickness was obtained by varying the flux of air insufflated in the extruder head and the take up speed.

The prosthesis prepared with one of the above described methods was at last wrapped with a Dacron® reinforcement, in the form of a stitch, to improve the mechanical properties both of the prosthesis itself and of the duct to be rigenerated.

The activation of the prosthesis was performed with an Ar cold plasma, by operating at 50 W, 3 minutes, at the temperature of 20°C. The activated prosthesis was then sterilised and seeded with endothelial cells, with the methodology described in the example 9, before being implanted.

The invention as it is exemplified above is susceptible to numerous modifications and variations all falling within the ambit of protection of the following claims.

## Claims

1. A process for the surface treatment of biocompatible and bioabsorbable aliphatic polyesters selected from a group formed by homo and copolymers derived from L-lactide, D-lactide, D,L -lactide, racemic D,L-lactide, meso D,L-lactide, ε-caprolactone, glycolide, δ-valerolactone, p-dioxanone, preferably copolymers of L-lactide, D- lactide, racemic D,L-lactide, meso D,L-lactide with ε-caprolactone used for the production of biomedical products, **characterised in that** said process consists in carrying out an acid or base hydrolysis at temperatures comprised between 0°C and the softening temperature of the material and/or a treatment by cold plasma, said surface treatment increasing the surface hydrophilicity and lowering the wetting angle measured with water at 23°C in air and wherein the hydrolytic treatment does not induce any reduction in the mechanical properties of the products.

2. A process as claimed in claim 1, **characterised in that** said aliphatic polyesters comprise 50/50 poly(L-lactide-co-e-caprolactone) and the hydrolysis is conducted at temperatures comprised between 5°C and 80°C, preferably between 30°C and 50°C.

3. A process as claimed in anyone of the claims from 1 or 2, **characterised in that** said hydrolysis treatment is conducted in the presence of an aqueous solution of a base.

4. A process as claimed in claim 3 **characterised in that** said base is a strong alkali.

5. A process as claimed in claim 4, **characterised in that** said strong alkali is selected from a group formed by NaOH, KOH, Na₂CO₃, LiOH, Ca(OH)₂.

6. A process as claimed in claim 3, **characterised in that** said base is a weak base.

7. A process as claimed in claim 6, **characterised in that** said weak base is NH₄OH.

8. A process as claimed in claim 6 or 7, **characterised in that in that** said weak base comprises at least one amine.

9. A process as claimed in claim 8, **characterised in that** said amine comprises at least one amine selected from a group formed by methylamine, ethylamine, diethylamine and dimethylamine.

10. A process as claimed in anyone of claims from 1 to 3, **characterised in that** said hydrolysis treatment is conducted in the presence of an acqueous solution of an acid.

11. A process as claimed in claim 10, **characterised in that** said acid is selected from a group formed by HCl, HClO₃, HClO₄, H₂SO₃, H₂SO₄, H₃PO₃, H₃PO₄, HI, HIO₃, HBr, lactic acid, glicolic acid.

12. A process as claimed in any one of claims from 1 to 11, **characterised in that** the surface hydrolysis is followed by washing for removing any trace of acid or base.

13. A process as claimed in claim 1, **characterised in that** said plasma treatment is carried out in a presence of a reactive gas for a formation of surface activations of oxygenate type.

14. A process as claimed in claim 13, **characterised in that** said reactive gas is air and said surface activations of oxygenate type are -OH, -CHO, -COOH.

15. A process as claimed in claim 13, **characterised in that** said plasma treatment is carried out in a presence of argon.

16. A process as claimed in claim 13, **characterised in that** said plasma treatment is carried out with oxygen.

17. A process as claimed in claim 13, **characterised in that** said plasma treatment is carried out with air.

18. A process as claimed in any one of claims from 1 to 17, **characterised in that** the hydrolysis or the plasma treatment is followed by a phase of induction of further chemical modifications.

19. A process as claimed in claim 18, **characterised in that** -COONa groups generated by base hydrolysis are converted into -COOH groups by means of treatment with strong mineral acids.

20. A process as claimed in claim 18, **characterised in that** a surface freeing of alcoholic and/or carboxylic and/or aldehydic groups by means of a hydrolysis process is followed by a chemical reaction by means of addition of a compound provided with an activated group or groups able to react with respective surface alcoholic and/or carboxylic and/or aldehydic groups.

21. A process as claimed in claim 20, **characterised in that** said compound comprises succinic anhydride, which causes a conversion of -OH groups into -O-CO-CH₂-CH₂-COOH groups.

22. A biocompatible and bioabsorbable aliphatic polyester selected from a group formed by homo and copolymers derived from L-lactide, D-lactide, D,L -lactide, racemic D,L-lactide, meso D,L-lactide, ε-caprolactone, glycolide, δ-valerolactone, p-dioxanone preferably copolymers of L-lactide, D- lactide, racemic D,L-lactide, meso D,L-lactide with ε-caprolactone, **characterized in that** the surface of said polymer is provided with a plurality of microporosities deriving from a hydrolytic treatment of the polyester, which increase the surface hydrophilicity and reduce the wetting angle measured with water at 23°C in air without inducing any reduction in the mechanical properties of said aliphatic polyester.

23. A biocompatible and bioabsorbable aliphatic polyester for biomedical use as claimed in claim 22, **characterised in that** said copolymers of L-lactide, D-lactide, D,L-lactide with ε-caprolactone are surface-activated with at least one of the groups selected from COONa, -COOH, OH.

24. A polyester according to any of claims 22 and 23, **characterised in that** its wetting angle measured with water at 23°C in air is about 30°.

25. A partially bioabsorbable vascular prosthesis, comprising a biocompatible and bioabsorbable aliphatic polyester as claimed in anyone of claims 22 to 24, the surface of which is activated by means of a process according to anyone of claims 1 to 21.

26. A prosthesis as claimed in claim 25, **characterised in that** it comprises a reinforcement sheath, advantageously made of Dacron®.

## Patentansprüche

1. Verfahren zur Oberflächenbehandlung biokompatibler und bioabsorbierbarer aliphatischer Polyester ausgewählt aus einer Gruppe, die aus Homo- und Copolymeren gebildet ist, stammend vom L-Lactid, D-Lactid, D,L-Lactid, optisch unaktivem D,L-Lactid, meso D,L-Lactid, ε-Caprolacton, Glycolid, δ-Valerolacton, p-Dioxanon, vorzugsweise Copolymere des L-Lactids, D-Lactids, optisch unaktiven D,L-Lactids, meso D,L-Lactids mit ε-Caprolacton, zur Verwendung der Herstellung biomedizinischer Produkte, **dadurch gekennzeichnet, dass** das Verfahren in der Durchführung einer Säure- oder Basenhydrolyse bei Temperaturen zwischen 0°C und der Erweichungstemperatur des Materials und/oder einer Behandlung mit kaltem Plasma besteht, wobei die Oberflächenbehandlung die Hydrophilie der Oberfläche vergrößert und den Benetzungswinkel gemessen mit Wasser bei 23°C an Luft verkleinert, und wobei die hydrolytische Behandlung keinerlei Verringerung der mechanischen Eigenschaften des Produkts bewirkt.

2. Vorrichtung nach Anspruch 1, **dadurch gekennzeichnet, dass** die apliphatischen Polyester 50/50 poly(L-Lactid-co-ε-Caprolacton) umfassen und die Hydrolyse bei Temperaturen zwischen 5°C und 80°C, vorzugsweise zwischen 30°C und 50°C, durchgeführt wird.

3. Verfahren nach einem der Ansprüche 1 oder 2, **dadurch gekennzeichnet, dass** die Hydrolysebehandlung in der Anwesenheit einer wässrigen Lösung einer Base durchgeführt wird.

4. Verfahren nach Anspruch 3, **dadurch gekennzeichnet, dass** die Base ein starkes Alkali ist.

5. Verfahren nach Anspruch 4, **dadurch gekennzeichnet, dass** das starke Alkali ausgewählt ist aus einer Gruppe gebildet aus NaOH, KOH, Na₂CO₃, LiOH, Ca(OH)₂.

6. Verfahren nach Anspruch 3, **dadurch gekennzeichnet, dass** die Base eine schwache Base ist.

7. Verfahren nach Anspruch 6, **dadurch gekennzeichnet, dass** die schwache Base NH₄OH ist.

8. Verfahren nach Anspruch 6 oder 7, **dadurch gekennzeichnet, dass** die schwache Base wenigstens ein Amin umfasst..

9. Verfahren nach Anspruch 8, **dadurch gekennzeichnet, dass** das Amin wenigstens ein Amin umfasst, das aus einer Gruppe gebildet aus Methylamin, Ethylamin, Diethylamin und Dimethylamin ausgewählt ist.

10. Verfahren nach einem der Ansprüche 1 bis 3, **dadurch gekennzeichnet, dass** die Hydrolysebehandlung in der Anwesenheit einer wässrigen Lösung einer Säure durchgeführt wird.

11. Verfahren nach Anspruch 10, **dadurch gekennzeichnet, dass** die Säure aus einer Gruppe ausgewählt ist, gebildet aus HCl, HClO₃, HClO₄, H₂SO₃, H₂SO₄, H₃PO₃, H₃PO₄, HI, HIO₃, HBr, MilchSäure, Glykolsäure.

12. Verfahren nach einem der Ansprüche 1 bis 11, **dadurch gekennzeichnet, dass** auf die Oberflächenhydrolyse Waschen zur Entfernung jeglicher Spuren von Säure oder Base folgt.

13. Verfahren nach Anspruch 1, **dadurch gekennzeichnet, dass** die Plasmabehandlung in Anwesenheit eines reaktiven Gases zur Bildung von Oberflächenaktivierungen des Sauerstofftyps durchgeführt wird.

14. Verfahren nach Anspruch 13, **dadurch gekennzeichnet, dass** das reaktive Gas Luft ist und dass die Oberflächenaktivierungen des Sauerstofftyps -OH, -CHO, -COOH sind.

15. Verfahren nach Anspruch 13, **dadurch gekennzeichnet, dass** die Oberflächenbehandlung in Anwesenheit von Argon durchgeführt wird.

16. Verfahren nach Anspruch 13, **dadurch gekennzeichnet, dass** die Plasmabehandlung mit Sauerstoff durchgeführt wird.

17. Verfahren nach Anspruch 13, **dadurch gekennzeichnet, dass** die Plasmabehandlung mit Luft durchgeführt wird.

18. Verfahren nach einem der Ansprüche 1 bis 17, **dadurch gekennzeichnet, dass** auf die Hydrolyse- oder die Plasmabehandlung eine Phase des Einführens weiterer chemischer Modifikationen folgt.

19. Verfahren nach Anspruch 18, **dadurch gekennzeichnet, dass** durch basische Hydrolyse erzeugte -COONa Gruppen umgewandelt werden in -COOH Gruppen mittels einer Behandlung mit starken mineralischen Säuren.

20. Verfahren nach Anspruch 18, **dadurch gekennzeichnet, dass** einer Befreiung der Oberfläche von Alkohol- und/oder Carboxyund/oder Aldehyd-Gruppen mittels eines Hydrolyseverfahrens eine chemische Reaktion durch Zusatz einer Verbindung folgt, welche mit einer aktivierten Gruppe oder mit Gruppen versehen ist, die mit den betreffenden oberflächlichen Alkoholund/oder Carboxy- und/oder Aldehyd-Gruppen regieren können.

21. Verfahren nach Anspruch 20, **dadurch gekennzeichnet, dass** die Verbindung Bernsteinsäureanhydrid umfasst, das eine Umwandlung von -OH Gruppen in -O-CO-CH₂-CH₂-COOH Gruppen verursacht.

22. Biokompatibler und bioabsorbierbarer aliphatischer Polyester ausgewählt aus einer Gruppe, gebildet aus Homo- und Copolymeren, stammend vom L-Lactid, D-Lactid, D,L-Lactid, optisch unaktivem D,L-Lactid, meso D,L-Lactid, ε-Caprolacton, Glycolid, δ-Valerolacton, p-Dioxanon, vorzugsweise Copolymere des L-Lactids, D-Lactids, optisch unaktiven D,L-Lactids, meso D,L-Lactids mit ε-Caprolacton, **dadurch gekennzeichnet, dass** die Oberfläche des Polymers mit einer Vielzahl von Mirkoporositäten versehen ist, welche die Hydrophilie der Oberfläche erhöhen und den Benetzungswinkel gemessen mit Wasser bei 23°C an Luft verkleinern, ohne irgendeine Verringerung in den mechanischen Eigenschaften des aliphatischen Polyesters zu bewirken.

23. Biokompatibler und bioabsorbierbarer aliphatischer Polyester für den biomedizinischen Gebrauch nach Anspruch 22, **dadurch gekennzeichnet, dass** Copolymere des L-Lactids, D-Lactids, D,L-Lactids mit ε-Caprolacton oberflächenaktiviert sind mit wenigstens einer der Gruppen ausgewählt aus COONa, -COOH, OH.

24. Polyester nach einem der Ansprüche 22 und 23, **dadurch gekennzeichnet, dass** sein Benetzungswinkel gemessen mit Wasser bei 23°C an Luft ungefähr 30° ist.

25. Teilweise bioabsorbierbare vasculäre Prothese umfassend einen biokompartiblen und bioabsorbierbaren aliphatischen Polyester nach einem der Ansprüche 22 bis 24, wobei dessen Oberfläche mittels eines Verfahrens nach einem der Ansprüche 1 bis 21 aktiviert worden ist.

26. Prothese nach Anspruch 25, **dadurch gekennzeichnet, dass** sie eine Verstärkungsschicht, vorzugsweise hergestellt aus Dacron®, umfasst.

## Revendications

1. Procédé pour le traitement de surface de polyesters aliphatiques biocompatibles et bioabsorbables sélectionnés parmi le groupe formé par des homo- et copolymères dérivant de L-lactide, D-lactide, D,L-lactide, D,L-lactide racémique, D,L-lactide méso, ε-caprolactone, glycolide, δ-valérolactone, p-dioxanone, de préférence des copolymères de L-lactide, D-lactide, D,L-lactide racémique, D,L-lactide méso avec le ε-caprolactone utilisés pour la production de produits biomédicaux, **caractérisé en ce que** ledit procédé consiste à réaliser une hydrolyse acide ou basique à des températures comprises entre 0°C et la température de ramollissement du matériau et/ou un traitement par plasma froid, ledit traitement de surface augmentant les propriétés hydrophiles de la surface et abaissant l'angle de mouillage mesuré avec de l'eau dans de l'air à 23°C, le traitement hydrolytique ne provoquant pas de réduction des propriétés mécaniques des produits.

2. Procédé selon la revendication 1, **caractérisé en ce que** lesdits polyesters aliphatiques contiennent le 50/50 poly(L-lactide-co-ε-caprolactone) et l'hydrolyse est effectuée à des températures comprises entre 5°C et 80°C, de préférence entre 30°C et 50°C.

3. Procédé selon l'une quelconque des revendications 1 ou 2, **caractérisé en ce que** ledit traitement d'hydrolyse est effectué en présence d'une base en solution aqueuse.

4. Procédé selon la revendication 3, **caractérisé en ce que** ladite base est un alcali fort.

5. Procédé selon la revendication 4, **caractérisé en ce que** ledit alcali fort est sélectionné parmi un groupe formé par NaOH, KOH, Na₂CO₃, LiOH, Ca(OH)₂.

6. Procédé selon la revendication 3, **caractérisé en ce que** ladite base est une base faible.

7. Procédé selon la revendication 6, **caractérisé en ce que** ladite base faible est NH₄OH.

8. Procédé selon l'une des revendications 6 ou 7, **caractérisé en ce que** ladite base faible comprend au moins une amine.

9. Procédé selon la revendication 8, **caractérisé en ce que** ladite amine comprend au moins une amine sélectionnée parmi un groupe formé par la méthylamine, l'éthylamine, la diéthylamine et la diméthylamine.

10. Procédé selon l'une quelconque des revendications 1 à 3, **caractérisé en ce que** ledit traitement d'hydrolyse est effectué en présence d'un acide en solution aqueuse.

11. Procédé selon la revendication 10, **caractérisé en ce que** ledit acide est sélectionné parmi un groupe formé par HCl, HClO₃, HClO₄, H₂SO₃, H₂SO₄, H₃PO₃, H₃PO₄, HI, HIO₃, HBr, l'acide lactique, l'acide glycolique.

12. Procédé selon l'une quelconque des revendications 1 à 11, **caractérisé en ce que** l'hydrolyse de surface est suivie par un lavage pour éliminer les traces d'acide ou de base.

13. Procédé selon la revendication 1, **caractérisé en ce que** ledit traitement par plasma est réalisé en présence d'un gaz réactif pour former des activations de surface de type oxygénées.

14. Procédé selon la revendication 13, **caractérisé en ce que** ledit gaz réactif est de l'air et lesdites activations de surface de type oxygénées sont -OH, -CHO, -COOH.

15. Procédé selon la revendication 13, **caractérisé en ce que** ledit traitement par plasma est réalisé en présence d'argon.

16. Procédé selon la revendication 13, **caractérisé en ce que** ledit traitement par plasma est réalisé avec de l'oxygène.

17. Procédé selon la revendication 13, **caractérisé en ce que** ledit traitement par plasma est réalisé avec de l'air.

18. Procédé selon l'une quelconque des revendications 1 à 17, **caractérisé en ce que** le traitement d'hydrolyse ou par plasma est suivi par une phase d'induction de modifications chimiques supplémentaires.

19. Procédé selon la revendication 18, **caractérisé en ce que** les groupes -COONa générés par l'hydrolyse basique sont convertis en groupe -COOH au moyen de traitement avec des acides minéraux forts.

20. Procédé selon la revendication 18, **caractérisé en ce que** une libération de surface de groupes alcooliques et/ou carboxyliques et/ou aldéhydiques au moyen d'un procédé d'hydrolyse est suivie par une réaction chimique par adition d'un composé provenant d'un groupe activé ou de groupes capables de réagir avec respectivement les groupes alcooliques et/ou cabroxyliques et/ou aldéhydiques de surface.

21. Procédé selon la revendication 20, **caractérisé en ce que** ledit composé comprend l'anhydride succinique, qui provoque une conversion des groupes -OH en des groupes -O-CO-CH₂-CH₂-COOH.

22. Polyester aliphatique biocompatible et bioabsorbable sélectionné parmi un groupe formé par des homo- et copolymères dérivant de L-lactide, D-lactide, D,L-lactide, D,L-lactide racémique, D,L-lactide méso, ε-caprolactone, glycolide, δ-valérolactone, p-dioxanone, de préférence des copolymères de L-lactide, D-lactide, D,L-lactide racémique, D,L-lactide méso avec le ε-caprolactone, **caractérisé en ce que** la surface dudit polymère est munie d'une pluralité de microporosités provenant d'un traitement hydrolytique du polymère, qui augmente les caractéristiques hydrophiles de la surface et réduit l'angle de mouillage mesuré avec l'eau dans de l'air à 23°C sans induire de réduction des propriétés mécaniques dudit polyester aliphatique.

23. Polyester aliphatique biocompatible et bioabsorbable pour des applications biomédicales selon la revendication 22, **caractérisé en ce que** lesdits copolymères de L-lactide, D-lactide, D,L-lactide avec du ε-caprolactone ont une surface activée avec au moins un des groupes sélectionnés parmi -COONa, -COOH, -OH.

24. Polyester selon l'une quelconque des revendications 22 et 23, **caractérisé en ce que** son angle de mouillage mesuré avec l'eau dans l'air à 23°C est d'environ 30°.

25. Prothèse vasculaire partiellement absorbable, comprenant un polyester aliphatique biocompatible et bioabsorbable selon l'une quelconque des revendications 22 à 24, la surface de celui-ci étant activée au moyen d'un procédé selon l'une quelconque des revendications 1 à 21.

26. Une prothèse selon la revendication 25, **caractérisée en ce que** elle comprend une gaine de renforcement, avantageusement faite en Dacron®.
